(19) Europäisches Patentamt
European Patent Office
Office européen des brevets

(11) Numéro de publication : **0 444 983 B1**

# (12) FASCICULE DE BREVET EUROPEEN

(45) Date de publication du fascicule du brevet : **14.07.93 Bulletin 93/28**

(51) Int. Cl.$^5$ : **A61K 7/00**

(21) Numéro de dépôt : **91400263.9**

(22) Date de dépôt : **05.02.91**

## (54) Procédé de fabrication d'une composition cosmétique capillaire, composition et procédé de traitement cosmétique.

(30) Priorité : **23.02.90 FR 9002301**

(43) Date de publication de la demande : **04.09.91 Bulletin 91/36**

(45) Mention de la délivrance du brevet : **14.07.93 Bulletin 93/28**

(84) Etats contractants désignés : **AT BE CH DE DK ES FR GB GR IT LI NL SE**

(56) Documents cités :
**EP-A- 0 155 806**
**FR-A- 2 315 991**
**FR-A- 2 597 367**

(73) Titulaire : **L'OREAL**
**14, Rue Royale**
**F-75008 Paris (FR)**

(72) Inventeur : **Hansenne-Richoux, Isabelle**
**19, rue Boursault**
**F-75017 Paris (FR)**
Inventeur : **Audousset, Marie-Pascale**
**106, rue Baudin**
**F-92300 Levallois-Perret (FR)**

(74) Mandataire : **Peuscet, Jacques et al**
**Cabinet Peuscet 68, rue d'Hauteville**
**F-75010 Paris (FR)**



Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue Saint-Denis, 75001 PARIS

## Description

La présente invention concerne un procédé de fabrication d'une composition cosmétique pour application sur les cheveux, la composition cosmétique obtenue par ce procédé et un procédé de traitement cosmétique à l'aide de ladite composition.

Il est bien connu que les cheveux sont sensibilisés ou fragilisés à des degrés divers sous l'action des agents atmosphériques et/ou de certains traitements cosmétiques, tels que permanentes, teintures ou décolorations. Les cheveux sensibilisés ou fragilisés deviennent difficiles à démêler et à coiffer aussi bien à l'état mouillé qu'à l'état sec. De plus, ils sont rêches au toucher, n'ont plus un aspect lisse ou brillant et se chargent d'électricité statique.

On a donc cherché à appliquer sur la chevelure des compositions, qui permettent de remédier à ces différents problèmes.

Il est connu, de très longue date, d'utiliser des huiles et des corps gras pour redonner aux cheveux de la douceur et du brillant ; l'application de ces composés est généralement suivie d'un shampooing pour éliminer des cheveux l'excédent d'huile ou de corps gras. Cependant, l'utilisation des huiles et corps gras amollit et alourdit les cheveux et il est impossible d'obtenir par la suite une coiffure ayant de la tenue et du volume.

On a déjà proposé d'utiliser des compositions contenant des huiles de silicone. Celles-ci permettent d'obtenir des cheveux brillants, mais leur usage prolongé ou en grande quantité a l'inconvénient de donner aux cheveux un aspect gras.

On utilise couramment des compositions aqueuses contenant des agents tensioactifs cationiques, que l'on applique sur la chevelure et qu'on laisse agir pendant quelques minutes avant de rincer les cheveux. Les agents tensioactifs cationiques améliorent le démêlage et le coiffage, mais présentent des inconvénients: ils ont tendance à alourdir la chevelure et à lui donner un aspect gras. De plus, la chevelure a tendance à se resalir rapidement. Ces inconvénients sont d'autant plus accentués que les cheveux traités sont plus fins et plus sensibilisés.

Il est également connu d'utiliser des compositions contenant à la fois des agents tensioactifs cationiques et des huiles ou corps gras. Ces compositions peuvent avoir de bonnes propriétés cosmétiques et rhéologiques, mais elles ont tendance à former un dépôt sur les cheveux. Pour éviter la formation de ce dépôt, on a proposé d'utiliser des compositions contenant des silicones en plus des corps gras et des agents tensioactifs. En particulier, dans le document EP-A-0 155 806, on a décrit une composition aqueuse contenant des polydiméthylsiloxanes linéaires, des polydiméthylsiloxanes modifiés par au moins un polyalkylène-oxyde (diméthicone copolyols), un véhicule lipidique et un agent tensioactif cationique ; cette composition est préparée par simple mélange dans de l'eau chaude, refroidissement et agitation.

Il est, par ailleurs, connu que certains lipides amphiphiles sont susceptibles de former, par agitation en présence d'une phase aqueuse, une phase lamellaire lipidique hydratée conduisant à des vésicules ; le brevet français n° 2 315 991 décrit, en particulier, le cas où le lipide amphiphile est un lipide non-ionique. Ces vésicules sont constituées de couches concentriques de lipide(s) séparées par des couches de phase aqueuse interne. Le (les) lipide(s) non-ionique(s) utilisé(s) est (sont), de façon connue, des lipides amphiphiles d'origine naturelle ou synthétique comportant, par molécule, une (ou plusieurs) longue(s) chaîne(s) hydrocarbonée(s). Les nombreux procédés de fabrication des vésicules de lipides non-ioniques, sont bien connus. Dans un premier type de procédé (voir notamment US-A- 4 772 471), on dissout les lipides amphiphiles dans un solvant, puis on forme un film en évaporant le solvant; on met ensuite en contact sous agitation le film obtenu avec la phase aqueuse à encapsuler et on soumet le tout à une agitation énergique. Dans un second type de procédé (voir notamment FR-A-2 315 991), on évite l'emploi d'un solvant : dans une première étape, on met en contact le(s) lipide(s) amphiphile(s) non-ionique(s) fondu(s) (70 - 95°C par exemple) avec la phase aqueuse à encapsuler pour former une phase lamellaire hydratée, on poursuit l'addition de la phase aqueuse à encapsuler sous forte agitation jusqu'à formation des vésicules ; dans une seconde étape, on ajoute une phase aqueuse de dispersion, identique ou différente de la phase aqueuse à encapsuler en poursuivant l'agitation.

Selon la présente demande, on a trouvé que l'on améliore les caractéristiques de traitement des cheveux à l'aide d'une composition aqueuse contenant des silicones et des lipides, lorsqu'on utilise comme lipides des lipides amphiphiles non-ioniques susceptibles de former des vésicules et que l'on prépare la composition à l'aide d'un procédé connu de fabrication de vésicules non-ioniques, dans lequel on mélange le lipide amphiphile non-ionique et la silicone avant le processus de formation des vésicules.

On a vérifié, notamment par microscopie électronique, que la composition ainsi préparée contient des vésicules stables.

On a montré par des essais comparatifs que l'emploi de lipides non-ioniques amphiphiles susceptibles de former des vésicules permet d'obtenir de meilleurs résultats que l'emploi de lipides non-ioniques n'en formant pas et que le fait d'introduire la silicone dans le lipide amphiphile non-ionique avant le processus de formation

des vésicules permet également d'obtenir de meilleurs résultats que lorsque la silicone est introduite dans la phase aqueuse de dispersion des vésicules après formation de ceux-ci.

La présente invention a donc pour premier objet un procédé de fabrication d'une composition cosmétique pour application sur les cheveux contenant au moins un lipide, au moins une silicone et de l'eau, caractérisé par le fait que l'on mélange avec de l'eau au moins un lipide amphiphile non-ionique susceptible de former des vésicules avec au moins une silicone, que l'on soumet ce mélange à un procédé de formation de vésicules encapsulant une phase aqueuse, et que l'on effectue une dispersion du produit obtenu dans une phase aqueuse de dispersion.

De préférence la phase aqueuse de dispersion contient au moins un agent tensioactif cationique.

Le procédé de formation de vésicules utilisé est, de préférence, le procédé ne nécessitant pas de solvant, dans lequel on effectue une fusion du mélange de lipide(s) amphiphile(s) non-ionique(s) et de silicone(s), on introduit une phase aqueuse à encapsuler de façon à former une phase lamellaire hydratée, on poursuit l'addition de cette phase sous agitation énergique pour former des vésicules, puis on ajoute une phase aqueuse de dispersion.

On peut également utiliser le procédé dans lequel on dissout le(s) lipide(s) amphiphile(s) non-ionique(s) et la(les) silicone(s) dans un solvant organique, que, dans le récipient où est placée la solution ainsi obtenue, on évapore le solvant pour former, sur les parois dudit récipient, un film du mélange (lipide(s)/silicone(s)), que l'on ajoute dans ledit récipient la phase aqueuse à encapsuler, sous agitation énergique, jusqu'à formation des vésicules, et que l'on ajoute enfin une phase aqueuse de dispersion.

Le lipide amphiphile non-ionique utilisé selon la présente invention est, de préférence, choisi dans le groupe formé par les éthers et les esters de polyglycérol, linéaires ou ramifiés de formule (I) :

$$RO\left[C_3H_5(OH)-O\right]_{\bar{n}}-H \qquad (I)$$

- où $-C_3H_5(OH)O-$ est représenté par les structures suivantes, prises en mélange ou séparément:

$-CH_2CHOHCH_2O-$ ;

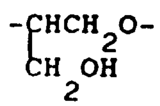 ; 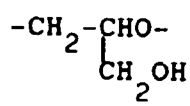 ;

- où $\bar{n}$ est une valeur statistique moyenne comprise entre 2 et 6 ;
- où R est :
  a) soit une chaîne aliphatique $R_1$ ou un reste $R_2CO$, $R_1$ étant un radical aliphatique, linéaire ou ramifié, en $C_{12}$-$C_{18}$ et $R_2$ étant un radical aliphatique, linéaire ou ramifié, en $C_{11}$-$C_{17}$ ;
  b) soit $R_3[O-C_2H_3(R_4)]$ où $-OC_2H_3(R_4)-$ est représenté par les structures suivantes, prises en mélange ou séparément :

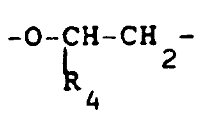 et 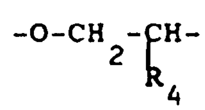 ;

$R_3$ étant un radical $R_1$ ou $R_2CO$ et

$R_4$ étant un radical $R_1$, $R_1$ et $R_2$ ayant les significations définies ci-dessus.

De façon connue, les lipide(s) amphiphile(s) non-ionique(s) constituant la phase lamellaire lipidique est (sont), de préférence, associé(s) à au moins un additif stabilisant destiné à modifier la perméabilité ou la charge superficielle des feuillets lipidiques. Selon l'invention, ces additifs sont plus particulièrement choisis parmi les stérols et/ou les stabilisants anioniques. Le stérol peut être avantageusement le cholestérol ou le bêta-sitostérol ; on utilise, de préférence, un mélange poids pour poids de lipide(s) amphiphile(s) non-ionique(s) et de cholestérol ou un mélange contenant moins de 50 % en poids de cholestérol. Le stabilisant anionique est avantageusement choisi parmi les sels monosodiques ou disodiques de glutamate d'acyle, le radical acyle étant en $C_{14}$ - $C_{22}$ tels que le sel monosodique du glutamate de stéaryle, les sels disodiques des glutamates

de cocoyle, de stéaryle ou de mélange de radicaux acylés du coprah et du suif ; et les esters phosphoriques d'alcools gras en $C_{12}$ - $C_{16}$; les stabilisants anioniques sont associés au(x) lipide(s) amphiphile(s) non-ionique(s) en quantité ne dépassant pas généralement 12 % en poids par rapport au poids du (des) lipide(s) amphiphile(s) non-ionique(s). De façon connue, on peut ajouter aux lipides amphiphiles non-ioniques à la fois un stérol et un stabilisant anionique.

La (les) silicone(s) mélangée(s) aux lipides amphiphiles non-ioniques, selon la présente invention, est (sont), avantageusement, choisie(s) parmi les :

- polydiméthylsiloxanes et leurs mélanges avec un triméthylsiloxysilicate ;
- polydiméthylsiloxanes modifiés par des groupes hydroxyle en bout de chaîne ;
- polydiméthylsiloxanes modifiés par des groupements alcoxy en $C_{12}$ - $C_{22}$ ;
- polydiméthylsiloxanes modifiés par des groupements polyoxyalkylénés, le radical alkylène étant en $C_2$ ou $C_3$ ;
- polydiméthylsiloxanes modifiés par des radicaux acyloxyalkyle dans lesquels le radical acyle est en $C_{12}$ - $C_{22}$ et le radical alkyle en $C_1$- $C_4$ ;
- polyméthylphénylsiloxanes, polyméthylalkyl ($C_1$- $C_{20}$)siloxanes ;
- polyméthyl [alkyl($C_1$- $C_4$ )aryl] siloxanes modifiés par des groupements alkyl($C_1$- $C_4$)amine ; et
- cyclopolysiloxanes.

Parmi les silicones avantageusement utilisées, on peut citer celles vendues sous les dénominations commerciales suivantes:

- "SILBIONE 47V500000" par la société "RHONE POULENC" (polydiméthylsiloxane ayant un poids moléculaire d'environ 250000);
- "SILBIONE huile 70045V5" par la société "RHONE POULENC" (décaméthylcyclopentasiloxane)";
- "FLUID DOW CORNING 593" par la société "DOW CORNING" ou "SS 4267 SILICONE FLUID" vendu par la société "GENERAL ELECTRIC CORP." (mélange de polydiméthylsiloxane et de triméthylsiloxysilicate);
- "SILICONE COPOLYMER F 555" par la société "S.W.S. SILICONES CORP." (stéaroxypolydiméthylsiloxane);
- "RHODORSIL HUILE 70633V30" par la société "RHONE POULENC" (polyméthylphénylsiloxane);
- "GP 7100 SILICONE FLUID" par la société "GENESEE POLYMERS CORP." (polyméthyl(alkylaryl)-siloxane modifié par des groupements alkylamine);
- "VOLATILE SILICONE FZ 3109" vendu par la société "UNION CARBIDE" (tétraméthyltétraoctylcyclotétrasiloxane).

Les silicones utilisées peuvent être sous forme d'huiles, de gommes ou de résines insolubles dans l'eau, volatiles ou non.

On utilise, de préférence, au moins une silicone non volatile telle qu'une huile de polydiméthylsiloxane hydroxylé en bout de chaîne ou une gomme de polyphénylméthylsiloxane, sous forme de solution dans au moins une huile de silicone volatile cyclique du type cyclométhicone.

On utilise, plus particulièrement, les solutions suivantes :

- 15 % en poids d'un polydiméthylsiloxane non volatil tel que celui vendu sous la dénomination commerciale "SILBIONE 47V500000" par la société "RHONE POULENC" en solution dans 85 % en poids de décaméthylcyclopentasiloxane tel que celui vendu sous la dénomination commerciale "VOLATILE SILICONE 7158" par la société "UNION CARBIDE";
- 13 % en poids d'un mélange de polydiméthylsiloxanes non volatils hydroxylés en bout de chaîne tel que celui vendu sous la dénomination commerciale "Q2 1401" par la société "DOW CORNING" en solution dans 87 % en poids d'un mélange de décaméthylcyclopentasiloxane et d'octaméthylcyclotétrasiloxane;
- 15 % en poids d'une gomme de phénylméthylsiloxane non volatile ayant un poids moléculaire de 600000 environ en solution dans 85 % en poids d'un mélange de décaméthylcyclopentasiloxane et d'octaméthylcyclotétrasiloxane.

L'agent tensioactif cationique éventuellement contenu selon l'invention dans la phase aqueuse de dispersion est généralement dispersé à chaud dans l'eau. Cette phase est ensuite mélangée à la dispersion contenant principalement le lipide non-ionique et la silicone. Le mélange est effectué soit à chaud, soit à la température ambiante. L'agent tensioactif est avantageusement un agent tensioactif insoluble dans l'eau à température ambiante. Il est, de préférence, au moins un dérivé d'ammonium quaternaire de formule II :

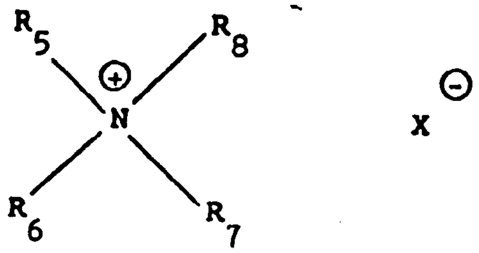

(II)

formule dans laquelle X est le chlore ou $CH_3SO_4$ et $R_5$ est un radical alkyle en $C_1$- $C_4$, de préférence le radical méthyle, et dans laquelle :

. ou bien $R_6$ et $R_7$ sont des radicaux alkyle en $C_1$- $C_4$, identiques ou différents de $R_5$ et entre eux, et $R_5$ est un radical alkyle en $C_{20}$- $C_{22}$;

. ou bien $R_6 = R_5$ et $R_7 = R_5$ = radical alkyle en $C_{18}$ ;

. ou bien $R_6$ désigne un radical (alkyle et/ou alkényle) amidoéthyle dans lequel le radical alkyle et/ou alkényle est en $C_{13}$- $C_{21}$ et dérive des acides gras du suif et $R_7$ et $R_8$ forment ensemble avec l'azote un hétérocycle 4,5-dihydroimidazole substitué, notamment en position 2, par un radical alkyle et/ou alkényle en $C_{13}$ - $C_{21}$.

L'agent tensioactif est plus particulièrement un chlorure de tétraalkylammonium de formule (II), dans lequel $R_5$, $R_5$ et $R_7$ sont des radicaux alkyle identiques en $C_1$ - $C_4$ , de préférence méthyle, et $R_8$ est un radical alkyle en $C_{20}$ - $C_{22}$ . On peut avantageusement utiliser le chlorure de béhényltriméthylammonium.

On peut également avantageusement utiliser le chlorure de distéaryldiméthylammonium, composé de formule (II) dans lequel $R_5 = R_6 = CH_3$ et $R_7 = R_8$ = alkyle en $C_{18}$.

Lorsque l'agent tensioactif est un méthyl-sulfate, il est avantageusement le composé de formule III:

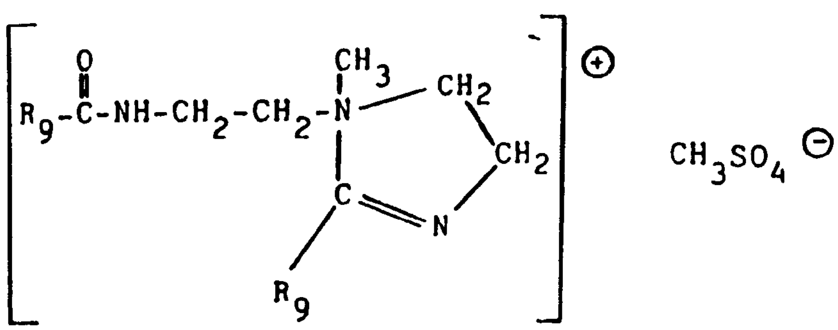

(III)

formule dans laquelle R désigne un mélange de radicaux alkényle et/ou alkyle en $C_{13}$- $C_{21}$ dérivé des acides gras du suif, par exemple le produit vendu sous la dénomination commerciale "REWOQUAT W 7500" par la société "REWO".

Selon la présente invention, on peut ajouter de façon connue au(x) lipide(s) amphiphile(s) non-ionique(s) et/ou à la (aux) silicone(s), avant la formation des vésicules, au moins un actif liposoluble cosmétique et/ou pharmaceutique, qui sera dans les feuillets lipidiques des vésicules. On peut également introduire, de façon connue, dans la phase aqueuse à encapsuler et/ou dans la phase aqueuse de dispersion, au moins un actif cosmétique et/ou pharmaceutique soluble dans l'eau et/ou au moins un additif. Parmi les actifs, on peut citer la vitamine A acide, l'acide linoléique, les tocophérols et les agents antichute ou de repousse des cheveux, les agents antipelliculaires, les rétinoïdes ou apparentés, les anti-inflammatoires, les antifongiques, les antiséborrhéiques, les filtres solaires ou analogues.

Parmi les additifs, on peut citer les conservateurs, les colorants, les parfums ou analogues. Dans la phase aqueuse de dispersion, on peut, en particulier, de façon connue, introduire un épaississant. Les agents épaississants sont plus particulièrement choisis parmi des dérivés cellulosiques comme l'hydroxyméthylcellulose, la carboxyméthylcellulose, l'hydroxybutylcellulose, l'hydroxypropylcellulose et plus particulièrement l'hydroxyéthylcellulose, tels que les produits vendus sous la dénomination commerciale "NATROSOL" (150,250) par la société "HERCULES" ou "CELLOSIZE" (QP et WP) par la société "UNION CARBIDE" ou "NATROSOL PLUS GRADE 330 CS" par la société "AQUALON", la méthylhydroxypropylcellulose, en particulier les produits vendus sous la dénomination "METHOCEL" (E,F,J,K) par la société "DOW CHEMICAL" ou des hétérobiopolysaccharides tels que par exemple les gommes de xanthane commercialisées sous les marques "KELTROL"

et "KELZAN" par la société "KELCO", "RHODOPOL" et "RHODIGEL" par la société "RHONE POULENC", ou "ACTIGUM" par la société "CECA/SATIA".

Ces agents épaississants peuvent être incorporés indifféremment dans des compositions contenant des silicosomes en présence ou non d'un tensioactif cationique.

Lorsque les compositions ne contiennent pas de tensioactif cationique, on peut également employer à titre d'agent épaississant les acides polyacryliques réticulés tels que les produits vendus sous la dénomination commerciale "CARBOPOL" par la société "GOODRICH", comme les Carbopols 910, 934, 934P, 940, 941, 1342.

Il est également possible d'introduire de façon connue dans la phase aqueuse de dispersion une substance non-miscible à l'eau telle qu'une huile.

La présente invention a pour deuxième objet une composition obtenue par le procédé ci-dessus défini, comprenant au moins une silicone et au moins un lipide amphiphile non-ionique sous forme de vésicules dispersées dans une phase aqueuse de dispersion contenant également, de préférence, au moins un agent tensioactif cationique.

La composition selon l'invention contient avantageusement, calculés en poids par rapport au poids total de la composition :

- 1 à 10 % d'agent(s) tensioactif(s) cationique(s);
- 1,5 à 20 % de lipide(s) amphiphile(s) non-ionique(s);
- 0,5 à 10 % de silicone(s);

et de préférence:

- 1 à 7 % d'agent(s) tensioactif(s);
- 1,5 à 10 % de lipide(s) amphiphile(s) non-ionique(s);
- 1,5 à 5 % de silicone(s).

La composition selon l'invention se présente sous forme de crème ou de lotion.

Les compositions selon l'invention sont utilisées, de préférence, sous forme de produits à rincer, avant et plus particulièrement après un shampooing, avant et plus particulièrement après une coloration ou une décoloration, avant et plus particulièrement après une permanente ou un défrisage. On a constaté que ces compositions sont stables au cours du temps, même en présence d'agents tensioactifs cationiques, ce qui n'était aucunement prévisible par l'homme de métier, compte tenu notamment des indications contenues dans le brevet US-A-3 957 971 (colonne 11, ligne 1). En outre, ces compositions présentent un ensemble de propriétés cosmétiques avantageuses sur l'art antérieur et d'autant plus intéressant et surprenant que leur effet est immédiat, c'est-à-dire qu'il n'est pas nécessaire à l'utilisateur de laisser poser la composition avant de rincer, d'où un gain de temps très appréciable et une utilisation plus commode. Il demeure néanmoins possible de laisser la composition quelque temps avant de rincer et ceci sans modifier les bonnes propriétés obtenues.

Ces compositions ne graissent pas les cheveux, ne les ramollissent pas, les démêlent aisément et permettent un peignage facile à l'état humide ou sec.

Lesdites compositions confèrent, en outre, aux cheveux, des propriétés surprenantes telles qu'un lissage uniforme, une légèreté et une grande douceur de la racine à la pointe. On constate un effet surprenant d'individualisation des fibres capillaires et une diminution importante de l'électricité statique. Ces propriétés sont obtenues sur les cheveux naturels ou peu sensibilisés lorsque la composition ne contient pas d'agent tensioactif cationique et également sur cheveux sensibilisés lorsque la composition contient un agent tensioactif cationique.

Ces compositions s'éliminent également très facilement au rinçage à l'eau.

La présente invention a pour troisième objet un procédé de traitement cosmétique de la chevelure, caractérisé par le fait que l'on applique sur celle-ci une quantité efficace de la composition selon l'invention, qu'éventuellement on peigne la chevelure et qu'enfin on rince ladite chevelure.

Selon ce traitement, on applique des quantités de compositions, en général, de l'ordre de 5 à 40 g par tête.

Les exemples donnés ci-dessous, à titre purement illustratif et non limitatif, permettront de mieux comprendre l'invention.

EXEMPLE 1

Dans une première étape, on prépare un premier constituant comprenant les vésicules. Pour cela, on fond, à une température de 95°C, un mélange de 10,8 g de lipides non-ioniques de formule:

$$C_{16}H_{33}-O-\left[C_3H_5(OH)O\right]_{\bar{n}}-H$$

où $\bar{n}$ est une valeur statistique moyenne égale à 3, et $C_3H_5(OH)O$ est représenté par le mélange des structures suivantes:

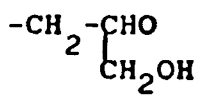 et 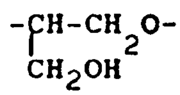

avec 10,8 g de cholestérol et 1,2 g de sel monosodique du glutamate de stéaryle vendu sous la dénomination commerciale "ACYLGLUTAMATE HS 11" par la société "AJINOMOTO". Puis au mélange fondu, on ajoute 28,6 g d'un mélange contenant 13 % en poids d'un polydiméthylsiloxane hydroxylé en bout de chaîne associé à 87 % en poids d'un mélange d'octaméthylcyclotétrasiloxane et de décaméthylcyclopentasiloxane vendu sous la dénomination commerciale "Q2 1401" par la société "DOW CORNING" et, simultanément, on soumet le mélange à une agitation douce pendant 5 min. (temps nécessaire à une parfaite homogénéisation).

On introduit dans le mélange fondu 103 g d'eau portée à 90°C contenant un conservateur et l'on mélange pendant environ 5 min. A la phase ainsi obtenue, on ajoute 154 g d'eau à 20°C et on agite le mélange pendant quelques minutes ; puis on complète par 461 g d'eau à 20°C et on affine le mélange par un passage dans un homogénéiseur haute pression à 500 bars de type "RANNIE".

Dans une seconde étape, on prépare un second constituant comprenant la phase aqueuse de dispersion ; pour ce faire, on dissout en 10 minutes à 80°C, 25,6 g d'une solution à 80 % de matière active (soit 22 g de matière active) d'un sel d'ammonium quaternaire vendu sous la dénomination commerciale "REWOQUAT W 7500" par la société "REWO" dans 141 g d'eau.

On mélange le premier et le second constituants ainsi obtenus et on homogénéise sous agitation douce.

Lorsque la température atteint environ 40°C, on ajoute le parfum, puis on complète à 1000 g par de l'eau à température ambiante et l'on agite doucement jusqu'à retour à température ambiante.

On obtient une composition, qui a l'aspect d'une crème. On a observé que cette composition était stable après trois mois à température ambiante en vérifiant par microscopie électronique la présence des vésicules.

On applique cette composition, à raison d'environ 15 g par tête, sur des cheveux sensibilisés, lavés et essorés. On rince abondamment à l'eau quelques secondes après la fin de l'application.

Les cheveux humides se démêlent aisément ; ils sont lisses et doux de la racine à la pointe. Après séchage, ils sont nerveux et se peignent très facilement ; ils ne sont pas électriques ; ils sont brillants, lisses et déliés sur toute leur longueur. La coiffure est légère et gonflante.

EXEMPLE 2

On reproduit l'exemple 1 en remplaçant le lipide non-ionique par celui de formule ci-après:

$$C_{15}H_{31}-CO-\left[O\ CH_2-CHOH-CH_2\right]_n-OH$$

formule dans laquelle n est égal à 2.

On obtient une composition stable, qui a les mêmes propriétés cosmétiques sur les cheveux que la composition de l'exemple 1.

EXEMPLE 3

On remplace dans l'exemple 1 le produit vendu sous la dénomination commerciale "REWOQUAT W 7500" par la société "REWO" par une quantité égale de chlorure de distéaryldiméthylammonium. On obtient une composition stable, qui a les mêmes propriétés cosmétiques sur les cheveux que la composition de l'exemple

1.

Dans les exemples 4 à 6 donnés ci-après, on opère comme dans l'exemple 1, sauf que l'on remplace la silicone vendue sous la dénomination commerciale "Q2 1401" par la société "DOW CORNING" par d'autres silicones en quantités égales.

EXEMPLE 4

La silicone est un mélange de 15 % en poids du produit vendu sous la dénomination commerciale "SILBIONE 47V500000" par la société "RHONE POULENC" dans 85 % en poids du produit vendu sous la dénomination commerciale "VOLATILE SILICONE 7158" par la société "UNION CARBIDE".

EXEMPLE 5

La silicone est un mélange de 15 % en poids d'un polydiméthylsiloxane (de poids moléculaire 600000) hydroxylé en bout de chaîne dans 85 % en poids d'un mélange de décaméthylcyclopentasiloxane et d'octaméthylcyclotétrasiloxane (50/50).

EXEMPLE 6

La silicone est un mélange de 15 % en poids de phénylméthylsiloxane (de poids moléculaire 600000) dans 85 % d'un mélange de décaméthylcyclopentasiloxane et d'octaméthylcyclotétrasiloxane (50/50).

Pour chacun des exemples 4 à 6 ci-dessus définis, on obtient des compositions stables, qui ont les mêmes propriétés cosmétiques que la composition de l'exemple 1.

EXEMPLE 7

On procède comme dans l'exemple 1 pour obtenir la composition suivante :

| | |
|---|---|
| - Composé non-ionique de formule IV (voir ci-dessous)...................... | 3,63 g |
| - Cholestérol............................. | 0,97 g |
| - Silicone vendue sous la dénomination commerciale "Q2 1401" par la société "DOW CORNING"............ | 2,86 g |
| - Agent tensioactif cationique vendu sous la dénomination commerciale "REWOQUAT W 7500" par la société "REWO"à 80% de matière active (MA) ...........(en MA) | 2,20 g |
| - Conservateur.........q.s. | |
| - Eau.................q.s.p.............. | 100,00 g |

La formule IV est la suivante :

$$C_{12}H_{25}\left[O\ C_2H_3\ (R_{10})\right]O\left[C_3H_5\ (OH)-O\right]_{\bar{n}}H$$

formule dans laquelle :

- O - $C_2H_3(R_{10})$- est constitué par un mélange de radicaux :

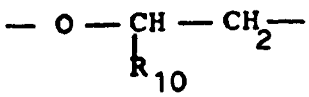 et 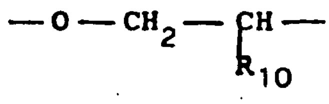

- $C_3H_5$ (OH) - O - est constitué par un mélange de radicaux :

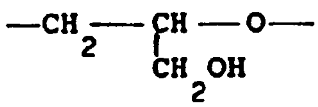 et 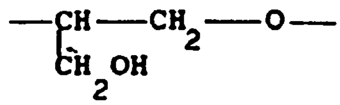

- $\bar{n} = 6$, et
- $R_{10}$ est un mélange des radicaux :
  $C_{14}H_{29}$- et $C_{16}H_{33}$-

<u>EXEMPLE 8</u> (Comparaison)

Dans cet exemple, on a comparé l'activité de deux compositions A et B contenant les mêmes quantités de la même silicone et ne contenant pas l'agent tensioactif cationique. La composition A, conforme à l'invention, contient des lipides amphiphiles nonioniques sous forme de vésicules et la composition B, non conforme à l'invention, contient des lipides non-ioniques amphiphiles qui ne forment pas de vésicules, les quantités de lipides A et B étant les mêmes.

Les formulations sont les suivantes:

| | Composition A | Composition B |
|---|---|---|
| Lipides amphiphiles selon l'invention (au total) : | 2,28 g | |
| sous forme d'un mélange de : | | |
| . Lipide non-ionique de l'exemple 1 ... | 1,08 g | |
| . Cholestérol ...... | 1,08 g | |
| . Sel monosodique du glutamate vendu sous la dénomina tion commerciale "ACYLGLUTAMATE HS 11" ........... | 0,12 g | |
| Lipides non-ioniques non conformes à l'in vention (au total) : | | 2,28 g |
| sous forme d'un mélange de : | | |
| . Stéarate de sorbitan | | |
| . Polysorbate 60 | | |
| . Mélange d'alcools cétylstéarylique et cétylstéary liques oxyéthylénés (15 moles d'oxyde d'éthylène) | | dans les proportions 0,23/0,15/1,90 |
| - Silicone vendue sous la dénomination commerciale "Q2 1401" par la société "DOW CORNING" | 2,86 g | 2,86 g |
| - Conservateur q.s. | | |
| - Eau ........ q.s.p. | 100 g | 100 g |

On a appliqué simultanément les compositions A et B à raison de 6 g par demi-tête sur des cheveux propres et mouillés. On rince l'ensemble de la chevelure à l'eau tiède, on peigne les cheveux et on les sèche. On compare ensuite l'état des cheveux sur les deux demi-têtes. Ces essais ont été effectués sur des cheveux naturels peu sensibilisés, sur cheveux permanentés et sur cheveux colorés permanentés.

Pour tous les cheveux traités, la composition A donne un effet de lissage de tenue et de gainage que l'on n'obtient pas avec la composition B. De plus, la composition A diminue le phénomène de formation d'électricité électrostatique et cet effet est conservé après un shampooing. Dans le cas des cheveux naturels peu sensibilisés, on obtient un effet de démêlage et de douceur et des cheveux légers avec la composition A que l'on n'obtient pas avec la composition B, laquelle au contraire les alourdit.

EXEMPLE 9 (Comparaison)

Dans cet exemple, on a comparé l'activité de deux compositions C et D ayant la même formulation que les compositions A et B, sauf que l'on a ajouté dans les deux compositions 2,2 g du tensioactif cationique vendu sous la dénomination commerciale "REWOQUAT W 7500" par la société "DOW CORNING".

Le processus d'application est le même dans les deux cas : on répartit, sur la totalité d'une chevelure ayant subi une permanente, 15 g de composition. On rince à l'eau tiède ; on peigne les cheveux et on les sèche.

On constate que la composition C est plus démêlante et communique aux cheveux plus de douceur et de brillance que la composition D, laquelle par ailleurs rend les cheveux gras en les alourdissant.

EXEMPLE 10

Dans cet exemple, on a comparé l'activité de deux compositions aqueuses E et F contenant les mêmes quantités des mêmes constituants lipide, silicone et tensioactif cationique :

- une composition E, conforme à l'invention, dans laquelle la silicone est mélangée au lipide amphiphile non-ionique avant formation des vésicules ;
- une composition F, non conforme à l'invention, dans laquelle la silicone est introduite dans la phase de dispersion.

Les compositions E et F correspondent à la formulation pondérale suivante:

- Lipide non-ionique de formule :

$$C_{16}H_{33}O - \left[ C_3H_5(OH)O \right]_{\bar{n}} - H$$

où $\bar{n}$ est une valeur statistique moyenne égale à 3 et $C_3H_5(OH)O$- est représenté par le mélange des structures suivantes :

$-CH_2-CHO-$ et $-CH-CH_2O-$
$CH_2OH$ $CH_2OH$ ......... 1,08 g

- Cholestérol............................ 1,08 g
- Sel monosodique du glutamate de stéaryle vendu sous la dénomination commerciale "ACYLGLUTAMATE HS 11" par la société "AJINOMOTO"............. 1,2 g
- Silicone vendue sous la dénomination commerciale "Q2 1401" par la société "DOW CORNING"......................... 2,86 g
- Agent tensioactif cationique vendu sous la dénomination commerciale "REWOQUAT W 7500" par la société "REWO" à 80 % de matière active (MA) ..........(en MA) 2,2 g
- Conservateur...........q.s.............
- Eau...................q.s.p........... 100 g

Le processus d'application est, dans les deux cas, celui défini à l'exemple 9. On constate que les cheveux traités avec la composition E selon l'invention présentent des propriétés cosmétiques supérieures quant au démêlage, douceur, brillance, gonflant, nervosité et légèreté et que les cheveux traités avec la composition F sont lourds et huileux à l'application.

EXEMPLE 11

La première étape est effectuée suivant l'exemple 1.

Dans une seconde étape, on prépare un second constituant comprenant les ingrédients de la phase aqueuse de dispersion : pour ce faire, on porte à une température de 80°C, 25,6 g du tensioactif cationique vendu sous la dénomination commerciale "REWOQUAT W 7500" par la société "REWO". On introduit dans le mélange fondu 51,20 g d'eau portée à 80°C et l'on mélange pendant environ 5 minutes. A la phase ainsi obtenue, on ajoute 76,8 g d'eau à 20°C. On agite le mélange quelques minutes, puis on ajoute 13 g d'eau à 20°C. On introduit le mélange dans un homogénéiseur haute pression à 500 bars de type "RANNIE".

On mélange le premier et le second constituants ainsi obtenus, sous agitation douce à température ambiante. On ajoute le parfum, puis on complète à 1000 g par de l'eau à température ambiante sous agitation douce.

Cette composition est plus particulièrement adaptée aux cheveux à tendance grasse. Elle rend ces cheveux légers, doux et brillants.

EXEMPLE 12

Dans une première étape, on prépare un premier constituant comprenant les vésicules. Pour cela, on fond, à une température de 95°C, un mélange de 10,9 g de lipide non-ioniques de formule:

$$C_{16}H_{33}-O-\left[C_3H_5(OH)O\right]_{\bar{n}}-H$$

où $\bar{n}$ est une valeur statistique moyenne égale à 3, et $C_3H_5(OH)O$ est représenté par le mélange des structures suivantes:

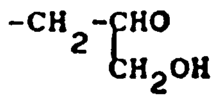 et 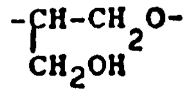

avec 10,9 g de cholestérol et 1,2 g de sel monosodique du glutamate de stéaryle vendu sous la dénomination commerciale "ACYLGLUTAMATE HS 11" par la société "AJINOMOTO". Puis au mélange fondu, on ajoute 0,4 g d'un mélange contenant 13 % en poids d'un polydiméthylsiloxane hydroxylé en bout de chaîne associé à 87 % en poids d'un mélange d'octaméthylcyclo tétrasiloxane et de décaméthylcyclopentasiloxane vendu sous la dénomination commerciale "Q2 1401" par la société "DOW CORNING", 42,5 g de décaméthylcyclopentasiloxane vendu sous la dénomination "SILBIONE huile 70045V5" par la société "RHONE POULENC", 7,5 g de polydiméthylsiloxane vendu sous la dénomination "SILBIONE 47V500000" par la société "RHONE POULENC" et simultanément, on soumet le mélange à une agitation douce pendant 5 min. (temps nécessaire à une parfaite homogénéisation).

On introduit dans le mélange fondu 147 g d'eau portée à 90°C contenant un conservateur et l'on mélange pendant environ 5 min. A la phase ainsi obtenue, on ajoute 220 g d'eau à 20°C et on agite le mélange pendant quelques minutes ; puis on complète par 300 g d'eau à 20°C et on affine le mélange par un passage dans un homogénéiseur haute pression à 500 bars de type "RANNIE".

Dans une seconde étape, on prépare un second constituant comprenant la phase aqueuse de dispersion ; pour ce faire, on dissout en 10 minutes à 80°C, 62,5 g d'une solution à 75 % de matière active (soit 46,9 g de matière active) d'un sel d'ammonium quaternaire vendu sous la dénomination commerciale "REWOQUAT W 7500" par la société "REWO" dans 141 g d'eau.

On mélange le premier et le second constituants ainsi obtenus et on homogénéise sous agitation douce. Puis au mélange obtenu, on ajoute comme agent épaississant 2,5 g d'hydroxyéthylcellulose modifiée par une chaîne alkyle vendue sous la dénomination "NATROSOL PLUS GRADE 330 CS" par la société "AQUALON" dissous dans 50 g d'eau à 80°C.

Lorsque la température atteint environ 40°C, on ajoute le parfum, puis on complète à 1000 g par de l'eau à température ambiante et l'on agite doucement jusqu'à retour à température ambiante.

On obtient une composition, qui a l'aspect d'une crème.

On applique cette composition, à raison d'environ 15 g par tête, sur des cheveux sensibilisés, lavés et essorés. On rince abondamment à l'eau quelques secondes après la fin de l'application.

Les cheveux humides se démêlent aisément ; ils sont lisses et doux de la racine à la pointe. Après séchage, ils sont nerveux et se peignent très facilement; ils ne sont pas électriques ; ils sont brillants, lisses et déliés sur toute leur longueur.

La coiffure est légère et gonflante.

## Revendications

1. Procédé de fabrication d'une composition cosmétique pour application sur les cheveux contenant au moins un lipide, au moins une silicone et de l'eau, caractérisé par le fait que l'on mélange avec de l'eau au moins un lipide amphiphile non-ionique susceptible de former des vésicules avec au moins une silicone, que l'on soumet ce mélange à un procédé de formation de vésicules encapsulant une phase aqueuse, et que l'on effectue une dispersion du produit obtenu dans une phase aqueuse de dispersion.

2. Procédé selon la revendication 1, caractérisé par le fait que la phase aqueuse de dispersion contient au moins un agent tensioactif cationique.

3. Procédé selon l'une des revendications 1 ou 2, caractérisé par le fait que l'on effectue une fusion du mélange de lipide(s) amphiphile(s) non-ionique(s) et de silicone(s), on introduit une phase aqueuse à encapsuler de façon à former une phase lamellaire hydratée, on poursuit l'addition de cette phase sous agitation énergique pour former des vésicules, puis on ajoute une phase aqueuse de dispersion.

4. Procédé selon l'une des revendications 1 ou 2, caractérisé par le fait que l'on dissout le(s) lipide(s) amphiphile(s) non-ionique(s) et la (les) silicone(s) dans un solvant organique, que, dans le récipient où est placée la solution ainsi obtenue, on évapore le solvant pour former, sur les parois dudit récipient, un film du mélange (lipide(s)/silicone(s)), que l'on ajoute dans ledit récipient la phase aqueuse à encapsuler, sous agitation énergique, jusqu'à formation des vésicules, et que l'on ajoute enfin une phase aqueuse de dispersion.

5. Procédé selon l'une des revendications 1 à 4, caractérisé par le fait que le lipide amphiphile non-ionique est choisi parmi les éthers et esters de polyglycérol linéaires ou ramifiés de formule (I):

$$RO\left[C_3H_5(OH)-O\right]_{\bar{n}}-H \qquad (I)$$

- où $-C_3H_5(OH)$ O- est représenté par les structures suivantes, prises en mélange ou séparément : $-CH_2CHOHCH_2O-$ ;

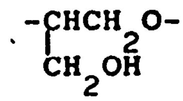 ; 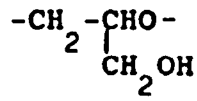 ;

- où $\bar{n}$ est une valeur statistique moyenne comprise entre 2 et 6 ;
- où R est :

  a) soit une chaîne aliphatique $R_1$ ou un reste $R_2CO$, $R_1$ étant un radical aliphatique, linéaire ou ramifié, en $C_{12}$-$C_{18}$, et $R_2$ étant un radical aliphatique, linéaire ou ramifié, en $C_{11}$- $C_{17}$ ;

  b) soit $R_3 \left[O-C_2H_3(R_4)\right]$

  où $-OC_2H_3(R_4)$- est représenté par les structures suivantes, prises en mélange ou séparément:

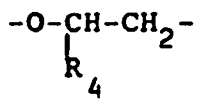 et 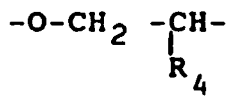 ;

  $R_3$ étant un radical $R_1$ ou $R_2CO$ et
  $R_4$ étant un radical $R_1$ , $R_1$ et $R_2$
  ayant les significations définies ci-dessus.

6. Procédé selon l'une des revendications 1 à 5, caractérisé par le fait que le(s) lipide(s) amphiphile(s) non-ionique(s) est (ou sont) associé(s) à au moins un additif stabilisant destiné à modifier la perméabilité ou la charge superficielle de la phase lamellaire lipidique hydratée.

7. Procédé selon l'une des revendications 1 à 6, caractérisé par le fait que la (ou les) silicone(s) mélangée(s) aux lipides sont choisies parmi les :
   - polydiméthylsiloxanes et leurs mélanges avec un triméthylsiloxysilicate ;
   - polydiméthylsiloxanes modifiés par des groupes hydroxyle en bout de chaînes ;
   - polydiméthylsiloxanes modifiés par des groupements alcoxy en $C_{12}$- $C_{22}$;
   - polydiméthylsiloxanes modifiés par des groupements polyoxyalkylène, le radical alkylène étant en $C_2$ ou $C_3$ ;

- polydiméthylsiloxanes modifiés par des radicaux acyloxyalkyle où le groupement acyle est en $C_{12}$-$C_{22}$ et le radical alkyle en $C_1$ - $C_4$ ;
- polyméthylphénylsiloxanes, polyméthylalkyl ($C_1$ - $C_{20}$)siloxanes ;
- polyméthyl[alkyl($C_1$ - $C_4$)aryl]siloxanes modifiés par des groupements alkyl ($C_1$ - $C_4$)amine ; et
- cyclopolysiloxanes.

**8.** Procédé selon l'une des revendications 1 à 7, caractérisé par le fait que l'on utilise au moins une silicone non volatile en solution dans au moins une huile de silicone volatile cyclique.

**9.** Procédé selon la revendication 8, caractérisé par le fait que l'agent tensioactif est au moins un dérivé d'ammonium quaternaire de formule II :

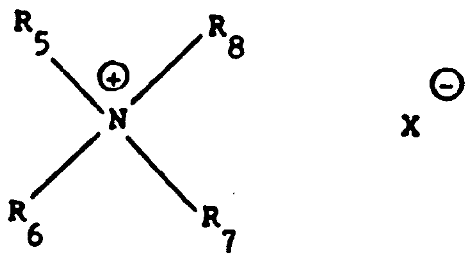

(II)

formule dans laquelle X est le chlore ou $CH_3SO_4$ et $R_5$ est un radical alkyle en $C_1$ - $C_4$, et dans laquelle :
. ou bien $R_6$ et $R_7$ sont des radicaux alkyle en $C_1$ - $C_4$ , identiques ou différents de $R_6$ et entre eux, et $R_6$ est un radical alkyle en $C_{20}$- $C_{22}$;
. ou bien $R_6$ = $R_6$ et $R_7$ = $R_8$ = radical alkyle en $C_{18}$;
. ou bien $R_6$ désigne un radical (alkyle et/ou alkényle)amidoéthyle dans lequel le radical alkyle et/ou alkényle est en $C_{13}$- $C_{21}$ et dérive des acides gras du suif ; et $R_7$ et $R_8$ forment ensemble avec l'azote un hétérocycle 4,5-dihydroimidazole substitué, en position 2, par un radical alkyle en $C_{13}$- $C_{21}$.

**10.** Procédé selon l'une des revendications 1 à 9, caractérisé par le fait que l'on ajoute au(x) lipide(s) amphiphile(s) non-ionique(s) et/ou au(x) silicone(s) au moins un actif liposoluble pharmaceutique et/ou cosmétique.

**11.** Procédé selon l'une des revendications 1 à 10, caractérisé par le fait que l'on introduit dans la phase aqueuse à encapsuler et/ou dans la phase aqueuse de dispersion au moins un actif cosmétique et/ou pharmaceutique soluble dans l'eau.

**12.** Procédé selon l'une des revendications 1 à 11, caractérisé par le fait que l'on introduit dans la phase aqueuse à encapsuler et/ou dans la phase de dispersion un additif pris dans le groupe formé par les conservateurs, les colorants ou les parfums.

**13.** Procédé selon l'une des revendications 1 à 12, caractérisé par le fait que l'on introduit dans la phase aqueuse de dispersion un agent épaississant.

**14.** Procédé selon l'une des revendications 1 à 13, caractérisé par le fait que l'on introduit dans la phase aqueuse de dispersion une substance non miscible à l'eau.

**15.** Composition obtenue par le procédé selon l'une des revendications 1 à 14, comprenant au moins une silicone et au moins un lipide amphiphile non-ionique sous forme de vésicules dispersées dans une phase aqueuse de dispersion.

**16.** Composition selon la revendication 15, caractérisée par le fait que la phase aqueuse de dispersion contient au moins un agent tensioactif cationique.

**17.** Composition selon la revendication 16, caractérisée par le fait qu'elle contient, calculés en poids par rapport au poids total de la composition :
- 1 à 10 % d'agent(s) tensioactif(s) cationique(s) ;
- 1,5 à 20 % de lipide(s) amphiphile(s) non-ionique(s) ;

- 0,5 à 10 % de silicone(s).

**18.** Composition selon la revendication 17, caractérisée par le fait qu'elle contient, calculés en poids par rapport au poids total de la composition :
- 1 à 7 % d'agent(s) tensioactif(s) ;
- 1,5 à 10 % de lipide(s) amphiphile(s) non-ionique(s) ;
- 1,5 à 5 % de silicone(s).

**19.** Procédé de traitement cosmétique de la chevelure, caractérisé par le fait que l'on applique sur celle-ci une quantité efficace de la composition selon l'une des revendications 15 à 18, qu'éventuellement on peigne la chevelure et qu'enfin on rince ladite chevelure.

**Patentansprüche**

**1.** Verfahren zur Herstellung eines kosmetischen Mittels zur Anwendung auf den Haaren, enthaltend wenigstens ein Lipid, wenigstens ein Silikon und Wasser, dadurch gekennzeichnet, daß man wenigstens ein nicht-ionisches, amphiphiles Lipid, das in der Lage ist, Vesikel zu bilden, mit wenigstens einem Silikon mit Wasser vermischt, daß man diese Mischung einem Verfahren zur Herstellung von Vesikeln, welche eine wäßrige Phase einkapseln, unterwirft und daß man das erhaltene Produkt in einer wäßrigen Dispersionsphase dispergiert.

**2.** Verfahren nach Anspruch 1, dadurch gekennzeichnet, daß die wäßrige Dispersionsphase wenigstens ein kationisches grenzflächenaktives Mittel enthält.

**3.** Verfahren nach einem der Ansprüche 1 oder 2, dadurch gekennzeichnet, daß man die Mischung aus nichtionischem, amphiphilem Lipid (nicht-ionischen, amphiphilen Lipiden) und Silikon (Silikonen) schmilzt, eine wäßrige einzukapselnde Phase einführt, so daß eine hydratisierte lamellare Phase gebildet wird, die Zugabe dieser Phase unter heftigem Bewegen fortsetzt, um Vesikel zu bilden und dann eine wäßrige Dispersionsphase zugibt.

**4.** Verfahren nach einem der Ansprüche 1 oder 2, dadurch gekennzeichnet, daß man das nicht-ionische amphiphile Lipid (die nicht-ionischen amphiphilen Lipide) und das Silikon (die Silikone) in einem organischen Lösungsmittel löst, daß man aus einem Behälter, in den die so erhaltene Lösung gegeben wurde, das Lösungsmittel verdampft, um an der Wand des Behälters einen Film aus der Mischung (Lipid(e)/Silikon(e)) zu bilden, daß man in den Behälter die wäßrige, einzukapselnde Phase unter heftigem Bewegen bis zur Bildung der Vesikel gibt und daß man schließlich eine wäßrige Dispersionsphase zugibt.

**5.** Verfahren nach einem der Ansprüche 1 bis 4, dadurch gekennzeichnet, daß das nicht-ionische, amphiphile Lipid ausgewählt ist unter linearen oder verzweigten Polyglycerinäthern oder -estern der Formel (I):

$$RO\left[C_3H_5(OH)-O\right]_{\bar{n}}-H \qquad (I)$$

- worin $-C_3H_5(OH)\ O-$ für die folgenden Reste, in Kombination oder einzeln, steht:
$-CH_2CHOHCH_2O-$ ;

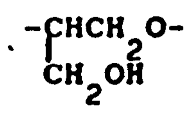 ; 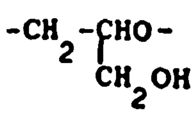 ;

- worin $\bar{n}$ einen statistischen Mittelwert zwischen 2 und 6 bedeutet;
- worin R bedeutet:

a) eine aliphatische Kette $R_1$ oder einen Rest $R_2CO$, wobei $R_1$ für einen linearen oder verzweigten, aliphatischen $C_{12}$-$C_{18}$-Rest steht und $R_2$ für einen linearen oder verzweigten, aliphatischen $C_{11}$-$C_{17}$-Rest steht;
b) $R_3$ [ $O-C_2H_3(R_4)$ ], worin $-OC_2H_3(R_4)-$ die folgenden Reste, in Kombination oder einzeln bedeutet:

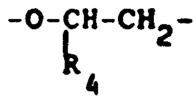 und 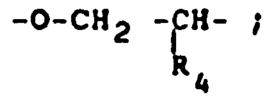

worin $R_3$ für einen Rest $R_1$ oder $R_2CO$ steht und $R_4$ für einen Rest $R_1$ steht, wobei $R_1$ und $R_2$ die obenangegebenen Bedeutungen besitzen.

6. Verfahren nach einem der Ansprüche 1 bis 5, dadurch gekennzeichnet, daß das nicht-ionische amphiphile Lipid (die nicht-ionischen amphiphilen Lipide) mit wenigstens einem stabilisierenden Additiv assoziiert ist (sind), das zur Modifikation der Permeabilität oder der Oberflächenladung der hydratisierten, lamellaren Lipidphase bestimmt ist.

7. Verfahren nach einem der Ansprüche 1 bis 6, dadurch gekennzeichnet, daß das (oder die) mit den Lipiden vermischte(n) Silikon(e) ausgewählt ist (sind) unter:
   - Polydimethylsiloxanen und ihren Mischungen mit einem Trimethylsiloxysilicat;
   - Polydimethylsiloxanen, die an den Kettenenden mit Hydroxygruppen modifiziert sind;
   - Polydimethylsiloxanen, die mit $C_{12}$-$C_{22}$-Alkoxygruppen modifiziert sind;
   - Polydimethylsiloxanen, die mit Polyoxyalkylengruppen modifiziert sind, wobei der Alkylenrest ein $C_2$- oder $C_3$-Rest ist;
   - Polydimethylsiloxanen, die mit Acyloxyalkylresten modifiziert sind, wobei die Acylgruppe ein $C_{12}$-$C_{22}$-Rest und der Alkylrest ein $C_1$-$C_4$-Rest ist;
   - Polymethylphenylsiloxanen, Polymethyl-($C_1$-$C_{20}$)-alkyl($C_1$-$C_{20}$)-siloxanen;
   - Polymethyl[($C_1$-$C_4$)Alkylaryl]siloxanen, die mit ($C_1$-$C_4$)Alkylamingruppen modifiziert sind; und
   - Cyclopolysiloxanen.

8. Verfahren nach einem der Ansprüche 1 bis 7, dadurch gekennzeichnet, daß man wenigstens ein nichtflüchtiges Silicon in Lösung in wenigstens einem flüchtigen, zyklischen Siliconöl verwendet.

9. Verfahren nach Anspruch 8, dadurch gekennzeichnet, daß das grenzflächenaktive Mittel wenigstens ein quartäres Ammoniumderivat der Formel II ist:

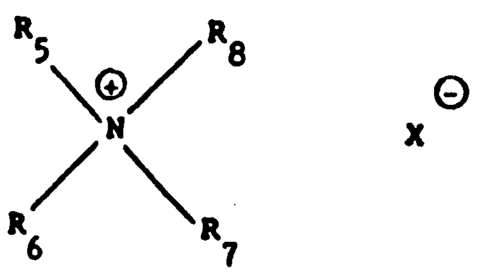 (II)

worin X für Chlor oder $CH_3SO_4$ steht und $R_5$ einen $C_1$-$C_4$-Alkylrest bedeutet und worin:
$R_6$ und $R_7$ entweder $C_1$-$C_4$-Alkylreste bedeuten, die zu $R_5$ und untereinander gleich oder verschieden sein können und Rg für einen $C_{20}$-$C_{22}$-Alkylrest steht;
oder $R_5 = R_5$ und $R_7 = R_5$ = ein $C_{18}$-Alkylrest;
oder $R_6$ einen (Alkyl- und/oder Alkenyl)amidoethylrest bedeutet, wobei der Alkyl- und/oder Alkenylrest ein $C_{13}$-$C_{21}$-Rest ist und sich von Talgfettsäuren ableitet;
und $R_7$ und $R_8$ zusammen mit dem Stickstoffatom einen 4,5-Dihydroimidazolheterozyklus bilden, der in 2-Stellung durch einen $C_{13}$-$C_{21}$-Alkylrest substituiert ist.

10. Verfahren nach einem der Ansprüche 1 bis 9, dadurch gekennzeichnet, daß man zu dem (den) nichtionischen, amphiphilen Lipid(en) und/oder dem (den) Silicon(en) wenigstens einen fettlöslichen, pharmazeutischen und/oder kosmetischen Wirkstoff gibt.

11. Verfahren nach einem der Ansprüche 1 bis 10, dadurch gekennzeichnet, daß man in die wäßrige einzukapselnde Phase und/oder in die wäßrige Dispersionsphase wenigstens einen wasserlöslichen, kosmetischen und/oder pharmazeutischen Wirkstoff gibt.

12. Verfahren nach einem der Ansprüche 1 bis 11, dadurch gekennzeichnet, daß man in die wäßrige einzukapselnde Phase und/oder in die Dispersionsphase ein Additiv aus der Gruppe der Konservierungsmittel, Farbstoffe oder Parfüms gibt.

13. Verfahren nach einem der Ansprüche 1 bis 12, dadurch gekennzeichnet, daß man in die wäßrige Dispersionsphase ein Verdickungsmittel gibt.

14. Verfahren nach einem der Ansprüche 1 bis 13, dadurch gekennzeichnet, daß man in die wäßrige Dispersionsphase eine mit Wasser nichtmischbare Substanz gibt.

15. Zusammensetzung erhältlich nach einem Verfahren gemäß einem der Ansprüche 1 bis 14, umfassend wenigstens ein Silikon und wenigstens ein nicht-ionisches amphiphiles Lipid in Form von Vesikeln, die in einer wäßrigen Dispersionsphase dispergiert sind.

16. Zusammensetzung nach Anspruch 15, dadurch gekennzeichnet, daß die wäßrige Dispersionsphase wenigstens ein kationisches, grenzflächenaktives Mittel enthält.

17. Zusammensetzung nach Anspruch 16, dadurch gekennzeichnet, daß sie, berechnet in Gewichtsanteilen bezogen auf das Gesamtgewicht der Zusammensetzung, enthält:
   - 1 bis 10% des kationischen, grenzflächenaktiven Mittels (der kationischen, grenzflächenaktiven Mittel);
   - 1,5 bis 20% des nicht-ionischen, amphiphilen Lipids (der nicht-ionischen, amphiphilen Lipide);
   - 0,5 bis 10% des Silikons (der Silikone).

18. Zusammensetzung nach Anspruch 17, dadurch gekennzeichnet, daß sie, berechnet in Gewichtsanteilen bezogen auf das Gesamtgewicht der Zusammensetzung, enthält:
   - 1 bis 7% des grenzflächenaktiven Mittels (der grenzflächenaktiven Mittel);
   - 1,5 bis 10% des nicht-ionischen, amphiphilen Lipids (der nicht-ionischen, amphiphilen Lipide);
   - 1,5 bis 5% des Silikons (der Silikone).

19. Verfahren zur kosmetischen Behandlung der Haare, dadurch gekennzeichnet, daß man auf die Haare eine wirksame Menge der Zusammensetzung nach einem der Ansprüche 15 bis 18 aufträgt, die Haare gegebenenfalls kämmt und sie schließlich spült.

## Claims

1. Process for manufacturing a cosmetic composition for application to the hair, containing at least one lipid, at least one silicone and water, characterised in that at least one nonionic amphiphilic lipid capable of forming vesicles with at least one silicone is mixed with water, in that this mixture is subjected to a process for forming vesicles encapsulating an aqueous phase, and in that the product obtained is dispersed in an aqueous dispersion phase.

2. Process according to Claim 1, characterised in that the aqueous dispersion phase contains at least one cationic surfactant.

3. Process according to one of Claims 1 and 2, characterised in that the mixture of nonionic amphiphilic lipid(s) and silicone(s) is melted, an aqueous phase to be encapsulated is introduced so as to form a hydrated lamellar phase, the addition of this phase is continued with vigorous agitation in order to form vesicles, and an aqueous dispersion phase is then added.

4. Process according to one of Claims 1 and 2, characterised in that the nonionic amphiphilic lipid(s) and

the silicone(s) are dissolved in an organic solvent, in that the solvent is evaporated off in the vessel in which the solution thereby obtained is placed in order to form a film of the (lipid(s)/silicone(s)) mixture on the walls of the said vessel, in that the aqueous phase to be encapsulated is added with vigorous agitation into the said vessel until vesicles are formed, and in that an aqueous dispersion phase is finally added.

**5.** Process according to one of Claims 1 to 4, characterised in that the nonionic amphiphilic lipid is chosen from the linear or branched ethers and esters of polyglycerol of formula (I):

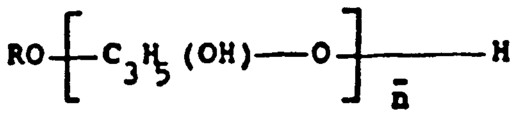 (I)

- where $-C_3H_5(OH)O-$ is represented by the following structures, taken mixed or separately:

$-CH_2CHOHCH_2O-$ ;

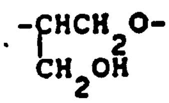 ; 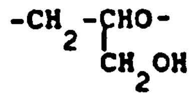 ;

- where $\bar{n}$ is an average statistical value between 2 and 6;
- where R is:

a) either an aliphatic chain $R_1$ or a residue $R_2CO$, $R_1$ being a linear or branched $C_{12}$-$C_{18}$ aliphatic radical, and $R_2$ being a linear or branched $C_{11}$-$C_{17}$, aliphatic radical;

b) or $R_3$ {$O-C_2H_3(R_4)$ }

where $-OC_2H_3(R_4)-$ is represented by the following structures, taken mixed or separately:

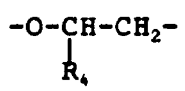 and 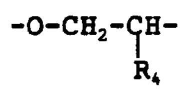 ;

$R_3$ being a radical $R_1$ or $R_2CO$ and $R_4$, being a radical $R_1$, $R_1$ and $R_2$ having the meanings defined above.

**6.** Process according to one of Claims 1 to 5, characterised in that the nonionic amphiphilic lipid(s) is/are combined with at least one stabilising additive intended for modifying the permeability or the surface charge of the hydrated lamellar lipid phase.

**7.** Process according to one of Claims 1 to 6, characterised in that the silicone(s) mixed with the lipids is/are chosen from:

- polydimethylsiloxanes and mixtures thereof with a trimethylsiloxy silicate;
- polydimethylsiloxanes modified by chain-end hydroxyl groups;
- polydimethylsiloxanes modified by $C_{12}$-$C_{22}$ alkoxy groups;
- polydimethylsiloxanes modified by polyoxyalkylene groups, the alkylene radical being a $C_2$ or $C_3$ radical;
- polydimethylsiloxanes modified by acyloxyalkyl radicals in which the acyl group is a $C_{12}$-$C_{22}$ group and the alkyl radical a $C_1$-$C_4$ radical;
- polymethylphenylsiloxanes, polymethyl($C_1$-$C_{20}$ alkyl)siloxanes;
- polymethyl[($C_1$-$C_4$ alkyl)aryl]siloxanes modified by ($C_1$-$C_4$ alkyl)amine groups; and
- cyclopolysiloxanes.

**8.** Process according to one of Claims 1 to 7, characterised in that at least one non-volatile silicone, dissolved in at least one cyclic volatile silicone oil, is used.

**9.** Process according to Claim 8, characterised in that the surfactant is at least one quaternary ammonium derivative of formula II:

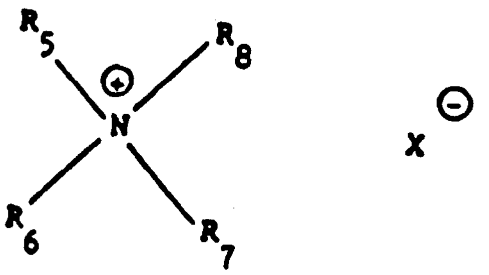

(II)

in which X is chlorine or $CH_3SO_4$ and $R_5$ is a $C_1$-$C_4$ alkyl radical, and in which:

. either $R_5$ and $R_7$ are $C_1$-$C_4$ alkyl radicals identical to or different from $R_5$ and each other, and $R_8$ is a $C_{20}$-$C_{22}$ alkyl radical;

. or $R_6 = R_5$ and $R_7 = R_8 = C_{18}$ alkyl radical;

. or $R_6$ denotes an (alkyl and/or alkenyl)amidoethyl radical in which the alkyl and/or alkenyl radical is a $C_{13}$-$C_{22}$ radical and is derived from tallow fatty acids; and $R_7$ and $R_8$, together with the nitrogen, form a 4,5-dihydroimidazole heterocycle substituted at position 2 with a $C_{13}$-$C_{21}$ alkyl radical.

**10.** Process according to one of Claims 1 to 9, characterised in that at least one pharmaceutical and/or cosmetic fat-soluble active substance is added to the nonionic amphiphilic lipid(s) and/or to the silicone(s).

**11.** Process according to one of Claims 1 to 10, characterised in that at least one water-soluble cosmetic and/or pharmaceutical active substance is introduced into the aqueous phase to be encapsulated and/or into the aqueous dispersion phase.

**12.** Process according to one of Claims 1 to 11, characterised in that an additive taken from the group composed of preservatives, colourings or perfumes is introduced into the aqueous phase to be encapsulated and/or into the dispersion phase.

**13.** Process according to one of Claims 1 to 12, characterised in that a thickening agent is introduced into the aqueous dispersion phase.

**14.** Process according to one of Claims 1 to 13, characterised in that a water-immiscible substance is introduced into the aqueous dispersion phase.

**15.** Composition obtained by the process according to one of Claims 1 to 14, comprising at least one silicone and at least one nonionic amphiphilic lipid in the form of vesicles dispersed in an aqueous dispersion phase.

**16.** Composition according to Claim 15, characterised in that the aqueous dispersion phase contains at least one cationic surfactant.

**17.** Composition according to Claim 16, characterised in that it contains, calculated by weight relative to the total weight of the composition:

- 1 to 10 % of cationic surfactant(a);
- 1.5 to 20 % of nonionic amphiphilic lipid(s);
- 0.5 to 10 % of silicone(s).

**18.** Composition according to Claim 17, characterised in that it contains, calculated by weight relative to the total weight of the composition:

- 1 to 7 % of surfactants(s);
- 1.5 to 10 % of nonionic amphiphilic lipid(s);
- 1.5 to 5 % of silicone(s).

**19.** Process for cosmetic treatment of the hair, characterised in that an effective amount of the composition according to one of Claims 15 to 18 is applied to the hair, in that the hair is combed where appropriate and in that the said hair is finally rinsed.